# EUROPEAN PATENT APPLICATION

(11) **EP 2 564 819 A1**
(43) Date of publication of application: **06.03.2013**
(21) Application number: 11179947.4
(22) Date of filing: 02.09.2011
(51) Int. Cl.: A61F 9/00

(54) **Intraocular injection device**

(71) Applicant: Sanofi-Aventis Deutschland GmbH, 65929 Frankfurt (DE)
(72) Inventor: The designation of the inventor has not yet been filed
(74) Representative: KEIL & SCHAAFHAUSEN Patentanwälte

(57) **Abstract**

Described is a displacement device (8) for displacing a conjunctiva of an eye (2) relative to a sclera. The displacement device (8) comprises a first leg (10), a second leg (11), a bar (12) hingedly coupled to the first leg (10) and the second leg (11) such that the bar (12) can move between a retracted position and an extended position, and a finger (13) coupled to the bar (12). The finger (13) is adapted to displace the conjunctiva relative to the sclera when the bar (12) is in the extended position.

## Description

### Background

A number of vision-threatening disorders or diseases of the eye need to deliver a drug (medicament or proteins or the like) by intraocular delivery (more specifically intravitreal delivery), especially when it is useful to deliver high concentrations of drugs. One such technique for intraocular delivery is accomplished by intraocular injection of the drug or capsules containing the drug directly into the vitreous body or by locating a device or capsule containing the drug in the vitreous with a syringe. Such an operation is used in particular for injection of compositions in the vitreous body of the eye in order to treat diseases affecting the retina or choroid, or ciliary body or the lens.

After delivery of drugs to the interior of the eye, such as the vitreous body, it is desirable that a point of entry of any drug delivery device closes and heals or seals as quickly and completely as possible after withdrawal of the drug delivery device. Sealing prevents reflux of the delivered drug, reduces internal eye pressure, heals the eye tissue affected (e.g. sclera), and prevents infections and other complications.

### Summary of the Invention

It is an object of the present invention to provide an apparatus for intraocular injection which could precisely be positioned in a desired zone of the eye and would allow for displacement of the superficial layer of the eye relative to the underlying layer prior to drug delivery and return of the superficial layer to its original position after drug delivery to allow for, e.g., occlusion of the point of entry of the drug delivery device.

In an exemplary embodiment, a displacement device for displacing a conjunctiva of an eye relative to a sclera comprises a first leg, a second leg, a bar hingedly coupled to the first leg and the second leg such that the bar can move between a retracted position and an extended position, and a finger coupled to the bar. The finger is adapted to displace the conjunctiva relative to the sclera when the bar is in the retracted position.

In an exemplary embodiment, the displacement device further comprises an adapter coupling the displacement device to a medicament delivery device. The adapter may be removably coupled to the medicament delivery device. The first leg may be shorter than the second leg.

In an exemplary embodiment, a medicament delivery device comprises a casing, a syringe having a needle disposed within and axially movable relative to the casing, and the displacement device, coupled to a distal end of the casing.

In an exemplary embodiment, movement of the syringe distally relative to the casing causes movement of the bar from the retracted position to the extended position. During movement of the syringe, a portion of the syringe engages the bar to cause movement of the bar from the retracted position to the extended position. The bar is in the extended position prior to the needle piercing the conjunctiva. The medicament delivery device may further comprise a spacer disposed on the distal end of the casing, the spacer including a contoured distal end adapted to contact the eye. The spacer may be telescopically coupled to the casing. At least a portion of the spacer may be transparent.

### Brief Description of the Drawings

Exemplary embodiments are described herein with reference to the schematic drawings in which:
Figure 1 shows a perspective view of parts of an exemplary embodiment of a medicament delivery device and a displacement device according to the present invention,
Figure 2 shows a partly cutaway view of an exemplary embodiment of a displacement device in a starting or finishing position,
Figure 3 shows a partly cutaway view of an exemplary embodiment of a displacement device in an intermediate (dosing) position,
Figure 4 shows a perspective view of an exemplary embodiment of a displacement device in the starting or finishing position and
Figure 5 shows an exemplary embodiment of a displacement device in the intermediate (dosing) position.

### Detailed Description

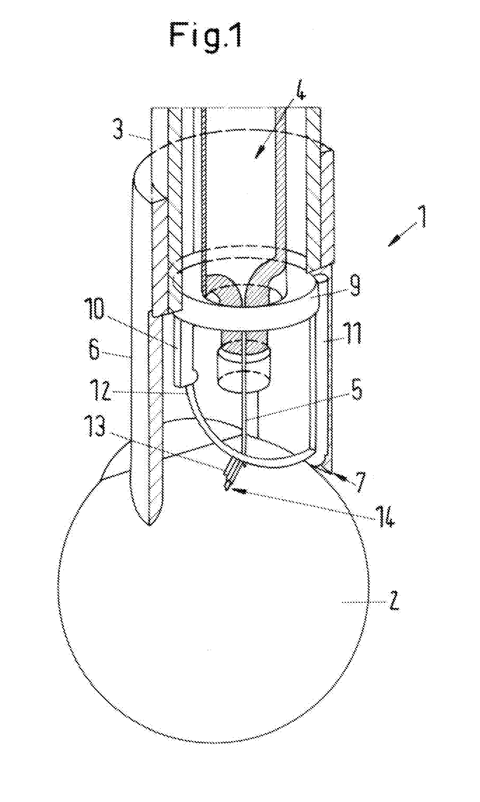

Figure 1 shows an exemplary embodiment of a medicament delivery device 1 according to the present invention. The delivery device 1 is provided with a casing 3 containing a syringe 4 which is movable within the casing 3. A distal end of the syringe includes a needle 5 for puncturing an injection site, e.g., an eye 2. The syringe 4 can be moved from a retracted position in which a distal tip of the needle 5 is spaced from the eye 2 into a distal position in which the needle 5 punctures the eye 2. In other exemplary embodiments, the casing 3 may include a cartridge of a medicament, and a distal end of the cartridge and/or the casing 3 may include a mechanism for detachably coupling to a replaceable needle assembly.

In an exemplary embodiment, a spacer 6 may be coupled to the distal end of the casing 3. The spacer 6 may telescopically fit on the casing 3 and move in conjunction with movement of the syringe 4, e.g., as the syringe 4 is advanced distally, the spacer 6 may advance distally, and vice-versa. Further, the spacer 6 may ensure that the distal tip of the needle 5 is not exposed past a distal end of the spacer 6. The distal end of the spacer 6 may include a foot portion 7 that is contoured to rest on an outer surface of the eye 2. The foot portion 7 may include a rounded edge adapted to align and position the delivery device 1 on the eye 2. In an exemplary embodiment, the spacer 6 may be transparent or windows may be formed therein to allow for visualization of the injection site.

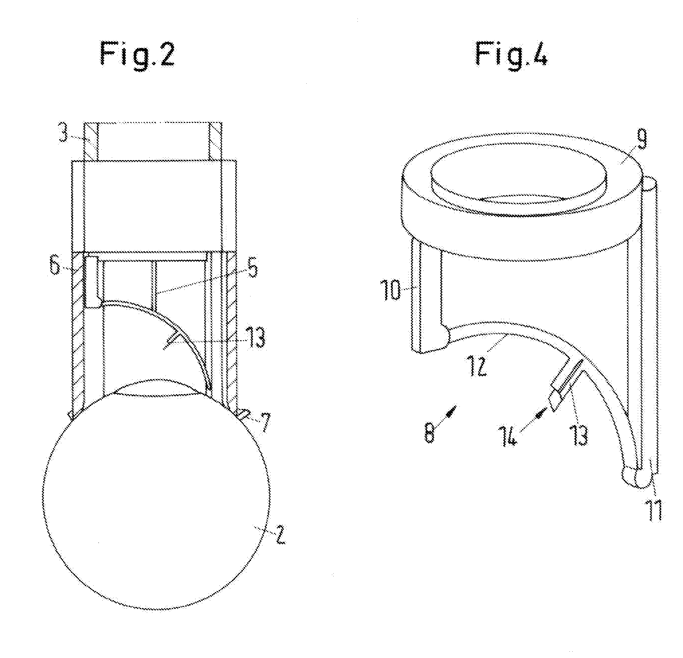

In an exemplary embodiment shown in Figure 4, a displacement device 8 is provided on a distal end of the casing 3. The displacement device 8 may be removably coupled to the distal end of the casing 3 (e.g., via an adapter 9) or integrally formed with the casing 3 or the spacer 6. Extending distally from the adapter 9 are a first leg 10 and a second leg 11. As can be seen from the exemplary embodiment in Figure 1, the legs 10, 11 are axial and parallel to a longitudinal axis of the casing 3. In an exemplary embodiment, the first leg 10 is shorter than second leg 11 such that when the second leg 11 contacts the outer surface of the eye 2, the first leg 10 is spaced from the outer surface of the eye 2.

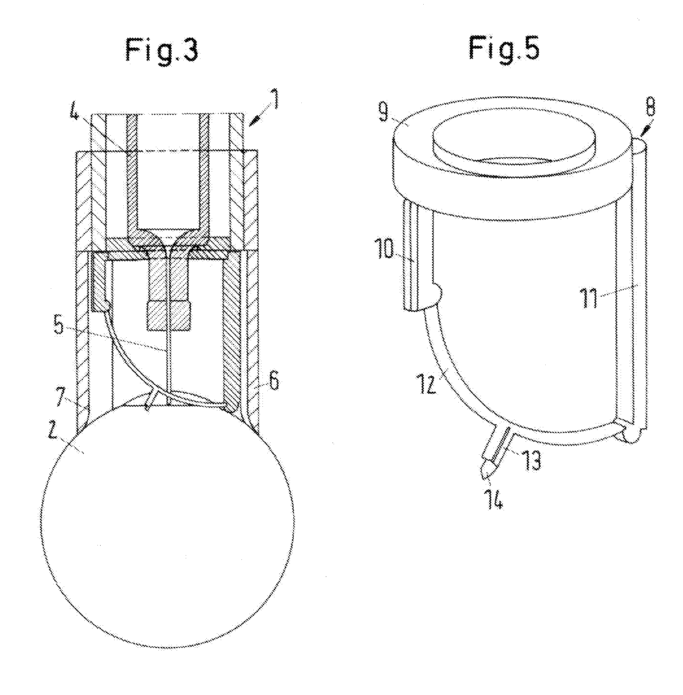

Referring back to the exemplary embodiment shown in Figure 4, a bar 12 is hingedly coupled to distal ends of the legs 10, 11. In an exemplary embodiment, the bar 12 is elastically deformable and can transition between a retracted position (e.g., shown in Figure 4) and an extended position (e.g., shown in Figure 5). The bar 12 may transition between the retracted position and the extended position by, for example, engagement with a distal end of the syringe 4. For example, a ledge may be formed on the bar 12 which engages the distal end of the syringe 4.

The bar 12 may include a finger 13 formed on a distal surface thereof. The finger 13 may include a tip 14 adapted to engage a superficial layer of the eye 2 when the bar 12 is in the extended position. The location of the finger 13 on the bar 12 may be fixed.

In operation, the delivery device 1 is placed on the eye 2, as shown in Figure 2. The foot portion 7 of the spacer 6 may be utilized to align the delivery device 1 over the injection site. The syringe 4 may be in the retracted position within the case 3, and the bar 12 may be in the retracted position such that the finger 13 does not engage the eye 2. During injection, the syringe 4 is advanced distally within the casing 3 and the distal end of the syringe 4 applies distal force to the bar 12 causing the bar 12 to transition to the extended position. Preferably, the bar 12 transitions to the extended position prior to the needle 5 piercing outer (conjunctiva) layer of the eye 2. When the bar 12 transitions to the extended position, the finger 13 engages the conjunctiva layer of the eye 2 and applies a laterally-directed force, displacing the conjunctiva relative to the sclera. After this displacement, the needle 5 pierces the eye 2 to deliver the medicament, as shown in Figure 3.

After the injection, the syringe 4 may be withdrawn into the casing 3, and the distal end of the syringe 4 may apply a proximally-directed force on the bar 12, forcing it into the retracted position. When the finger 13 is removed from the conjunctiva, the conjunctiva returns to its original position, and the injection holes in the conjunctiva and the sclera are not aligned.

In an exemplary embodiment, the displacement device 8 may be detached from the delivery device 1 and sterilized for reuse.

By displacing the conjunctiva relative to the sclera during the injection procedure, a punctured region (orifice) of the conjunctiva 51 is offset to the punctured region (orifice) of sclera when the conjunctiva is returned to its original position. Hence, the conjunctiva seals the orifice of the sclera, which may, for example, prevent reflux of the delivered drug, reduce the effects of the procedure on internal eye pressure, assist with the healing of the eye 2, and reduces the risk of infection.

Those of skill in the art will understand that modifications (additions and/or removals) of various components of the apparatuses, methods and/or systems and embodiments described herein may be made without departing from the full scope and spirit of the present invention, which encompass such modifications and any and all equivalents thereof.

## Claims

1. A displacement device (8) for displacing a conjunctiva of an eye (2) relative to a sclera, comprising:
a first leg (10);
a second leg (11);
a bar (12) hingedly coupled to the first leg (10) and the second leg (11) such that the bar (12) can move between a retracted position and an extended position; and
a finger (13) coupled to the bar (12), the finger (13) adapted to displace the conjunctiva relative to the sclera when the bar (12) is in the extended position.

2. The displacement device (8) according to claim 1, further comprising:
an adapter (9) coupling the displacement device (8) to a medicament delivery device (1).

3. The displacement device (8) according to claim 2, wherein the adapter (9) is removably coupled to the medicament delivery device (1).

4. The displacement device (8) according to any of the preceding claims, wherein the first leg (10) is shorter than the second leg (11).

5. A medicament delivery device (1), comprising:
a casing (3);
a syringe (4) disposed within the casing (3), the syringe (4) having a needle (5), the syringe (4) axially movable within the casing (3); and
the displacement device (8) according to any of the preceding claims,
wherein the displacement device (8) is coupled to a distal end of the casing (3).

6. The medicament delivery device (1) according to claim 5, wherein movement of the syringe (4) distally relative to the casing (3) causes movement of the bar (12) from the retracted position to the extended position.

7. The medicament delivery device (1) according to claim 6, wherein, during movement of the syringe (4), a portion of the syringe (4) engages the bar (12) to cause movement of the bar (12) from the retracted position to the extended position.

8. The medicament delivery device (1) according to claim 5, 6 or 7, wherein the bar (12) is in the extended position prior to the needle (5) piercing the conjunctiva.

9. The medicament delivery device (1) according to claim 5, 6, 7 or 8, further comprising:
a spacer (6) disposed on the distal end of the casing (3), the spacer (6) including a contoured distal end adapted to contact the eye (2).

10. The medicament delivery device (1) according to claim 9, wherein the spacer (6) is telescopically coupled to the casing (3).

11. The medicament delivery device (1) according to claim 9 or 10, wherein at least a portion of the spacer (6) is transparent.
